(19)

Europäisches
Patentamt
European
Patent Office
Office européen
des brevets

(11) **EP 1 797 855 A1**

(12) **EUROPEAN PATENT APPLICATION**

(43) Date of publication:
**20.06.2007 Bulletin 2007/25**

(51) Int Cl.:
*A61H 33/00* (2006.01)    *A61N 7/00* (2006.01)

(21) Application number: **05425883.5**

(22) Date of filing: **13.12.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(71) Applicant: **TEUCO GUZZINI S.p.A.
62010 Montelupone (IT)**

(72) Inventor: **Guzzini, Mauro
62019 Recanati (IT)**

(74) Representative: **Jorio, Paolo et al
Studio Torta S.r.l.
Via Viotti, 9
10121 Torino (IT)**

(54) **Method and apparatus for moisturising skin using ultrasound**

(57)     A cosmetic skin moisturizing treatment, whereby the user's skin is subjected to an "ultrasound dosage" ranging between 5 J/cm$^2$ and 400 J/cm$^2$, and preferably of 50 J/cm$^2$; the cosmetic treatment lasts from 2 to 10 minutes; and the ultrasonic waves range between 20 kHz and 50 kHz frequency.

Fig.1

EP 1 797 855 A1

# Description

The present invention relates to a cosmetic treatment and relative apparatus.

The cosmetic apparatus, for performing the cosmetic treatment which is the main object of the present invention, comprises a tub which is filled with a work fluid; a work fluid supply and drain device; and an ultrasound unit having a number of ultrasound generating devices.

Apparatuses comprising a tub equipped with devices for emitting ultrasonic waves in the fluid inside the tub are known.

Tubs of this sort are used for a wide range of applications.

Ultrasound bathing, in fact, has been proved effective in killing bacteria and fungi, and in hygiene in general.

Other ultrasound apparatuses, without a tub, are used for treating skin diseases and baldness.

Until now, however, ultrasound bathing has never been used for cosmetic skin moisturizing purposes.

The Applicant, in fact, has discovered, quite inventively and on the basis of original test results, that absorption of a work fluid (particularly water) by human skin is enhanced by subjecting the skin to bath treatment with "ultrasound dosages" of given values, the term "ultrasound dosage" being defined as the ratio between ultrasound energy and surface area.

Moreover, the best results, in terms of skin moisturization, have been found to be achieved with an ultrasonic wave frequency within a given range.

It is therefore the main object of the present invention to provide a cosmetic treatment designed to maximize moisturization of the user's skin.

The present invention will be described with reference to the accompanying Figures, in which:

Figure 1 shows an overall diagram of an apparatus for implementing the cosmetic treatment according to the present invention;
Figure 2 shows a first echogram of an untreated skin portion;
Figure 3 shows a second echogram of a skin portion treated using the cosmetic treatment which is the main object of the present invention;
Figure 4 shows a first dermatoscope image of an untreated skin portion; and
Figure 5 shows a second dermatoscope image of a skin portion treated with the cosmetic treatment which is the main object of the present invention.

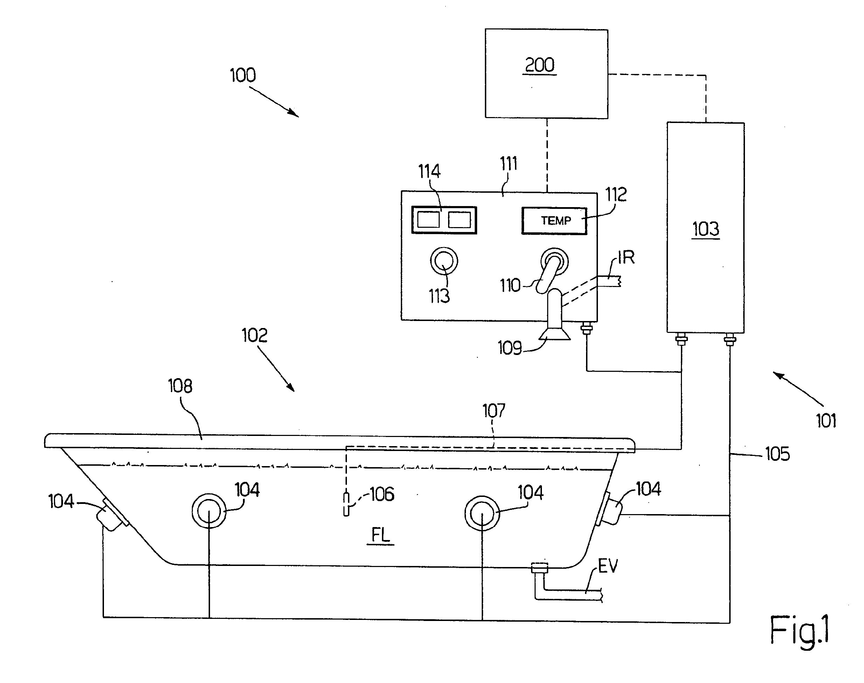

Number 100 in Figure 1 indicates a cosmetic apparatus for implementing a cosmetic treatment in accordance with the present invention.

Cosmetic apparatus 100 comprises, in known manner, an ultrasound generating and control system 101 fitted to an ultrasound application system 102.

Ultrasound generating and control system 101 in turn comprises an ultrasound generator 103 for generating a periodic electric signal; and a number of emitting transducers 104.

Ultrasound generator 103 is supplied with electrical energy by a mains (not shown), and emitting transducers 104 are connected electrically to the ultrasound generator by an electric cable 105.

System 101 may advantageously, though not necessarily, also comprise a receiving transducer 106, also connected electrically to ultrasound generator 103 by a respective electric cable 107.

Receiving transducer 106 provides for measuring the characteristics of the ultrasonic waves diffused in the work fluid, and for feedback of the readings.

Ultrasound application system 102 comprises a tub 108 containing a work fluid FL, and having a drain pipe EV for the used fluid FL.

More specifically, work fluid FL is water supplied by a tap 109 (in turn supplied by a water mains IR), but may be any fluid (containing water or not) deemed effective by cosmetic specialists.

The water supply from tap 109 is quantity and temperature controlled by a mixer 110 on a control panel 111.

Control panel 111 also comprises a first display 112 showing water temperature; a knob 113 for adjusting the energy intensity supplied by emitting transducers 104; and a second display 114 showing the intensity of the ultrasonic waves in tub 108.

Ultrasound generator 103 and control panel 111 are connected electrically in known manner to an electronic central control unit 200 for making the necessary adjustments and checks.

An apparatus 100 as described provides for achieving results similar, or even identical, to the test results described below.

## TEST RESULTS

### Foreword

The project deals with the possibility of employing ultrasonic waves of around 40 kHz to stimulate moisturization of skin immersed in a work fluid, particularly water.

Tests show moisturization in this way substantially affects the region between the stratum corneum and the dermis. Therefore, in any case the blood vessels are not affected by the present cosmetic treatment.

Testing comprised subjecting the hand skin of 92 healthy volunteers to low-frequency ultrasound fields (40 kHz) with a mean intensity of 50 and 125 mW/cm2.

Testing was conducted to compare skin exposed to ultrasonic waves in a water bath, with skin simply immersed in a water bath; and the difference in moisturization using ultrasonic waves in a water bath, and by applying the skin with medical moisturizing creams was assessed.

Testing was conducted using two tubs, each equipped with a transducer for producing a 40 kHz frequency ultrasound beam adjustable between 0 and 400 mW/cm2 peak intensity by means of a potentiometer, and with a duty cycle adjustable between 0 and 100% (see below).

Ninety-two healthy volunteers were subjected to the following test:

- both hands were immersed in two tubs containing water at about 30°C; only one of the two tubs had the ultrasound beam "on"; the transducer subjected the treated surface area to a mean intensity of 50 (in 48 cases) and 125 (in 44 cases) mW/cm2 at a distance of 1 cm from the transducer; the 1 cm skin-transducer distance was maintained constant using a spacer of plastic material;

- the treated area was marked out by skin boundary markers, and the skin was analyzed by measuring instruments and by dermatoscope and echograph imaging both before and after immersion in the tubs, to determine quantitative moisturization;

- the experiment was conducted in "double blind" conditions, i.e. none of the test subjects and none of the echograph operators knew which hand was subjected to ultrasonic waves, and which was simply immersed in water;

- exposure lasted 7 minutes at the above intensities; and the treated skin of the two different groups was then subjected to an "ultrasound dosage" of 21 J/cm2 and 52.5 J/cm2 respectively.

Test procedure

The back of the hand of each volunteer was marked with boundary markers indicating the treated (or simply exposed) area, the area in which to measure moisturization of the skin instrumentally, and the area for dermatoscope imaging.

The volunteer's skin was then subjected to pre-immersion measurements, and post-immersion measurements after 1 minute, 5 minutes, 15 minutes, and 60 minutes.

The following quantities were measured:

- TEWL (Trans Epidermal Water Loss);
- impedance (stratum corneum);
- skin temperature using a non-contact thermometer.

The measurements were made using Cortex Technology DERMALAB ® instruments.

The skin was also echo-scanned using a dedicated, high-resolution (50 MHz) dermatological echograph probe; the echograph used was a Cortex Technology DERMASCAN ®.

The skin was then scanned using a polarized-light, 20X dermatological microscope (a Scalar The-Scope® model).

Test data processing

The echograph images obtained were analyzed using dedicated software.

Special macros were produced in a developing environment (Media Cybernetics ImageProPlus®) capable of first-order statistical analysis of the texture of ROIs (Regions Of Interest) in the echograph images (ROI grey-level histogram analysis).

An ImageProPlus® subroutine was then written to evaluate numeric parameters characteristic of the cumulated grey-level profiles in the echograph images as a function of tissue depth.

Image analysis

Eight echograph images for each test volunteer were analyzed, four relative to a treated skin portion, and four relative to an untreated (simply immersed) skin portion.

This made a total of 736 echograph images. For each image, 15 numeric parameters were extracted, making a total of 11040 numeric values, which were then analyzed statistically.

The images analyzed were the pre-immersion images, and post-immersion images after 1 minute, 15 minutes, and 60 minutes.

A first image analysis approach was to evaluate the distances between tissue layers characterized by different acoustic impedances, and therefore identifiable by different grey levels.

For this purpose, it was opted to write a macro capable of converting grey levels to intensity profiles, which were then processed as described below.

The software, in fact, performs the following procedures:

1. opens the Dermascan image owner format;
2. calculates a cumulated image profile as a function of tissue depth;
3. calculates the integral of the 1st peak corresponding to the stratum corneum area;
4. calculates the centroid of the 1st peak;
5. calculates the mode (maximum) of the 1st peak;
6. calculates the integral of the 2nd peak corresponding to the dermis area;
7. calculates the centroid of the 2nd peak;
8. calculates the mode (maximum) of the 2nd peak; and
9. calculates the distances between centroids and between the mode of the 1st peak and the centroid of the second.

A second image analysis approach was to calculate statistical parameters characteristic of the images, by analyzing grey-level distribution in regions of interest centred on the stratum corneum of the images, using a so-called "texture analysis" approach.

A few synthetic numbers, significant of the "fine structure" of the image, are thus extracted from the distribution.

The microscope dermatoscopic images were also qualitatively inspected "visually" by three readers, to determine which was the ultrasound-treated skin portion image, and which image related to the skin portion simply immersed in water.

The results, gathered in an electronic sheet, were "chi-square" tested to determine their significance.

Results

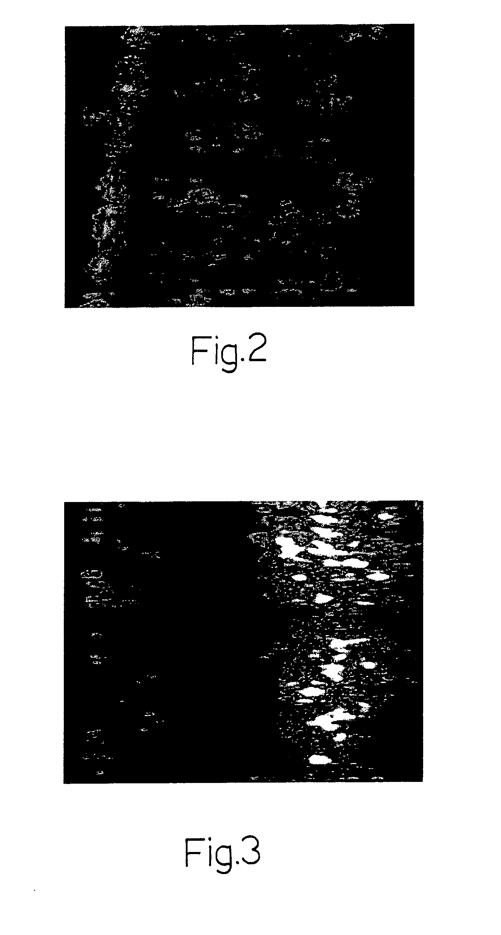

Analysis of the echograph images (Figures 2, 3) showed moisturization of the stratum corneum in practically all the volunteers treated (the dark areas in the echogram indicate liquid, and the light areas solid). As shown, in fact, by a comparison of Figures 2 and 3 (untreated and treated skin respectively), the dark areas in the subcorneum region are greatly enlarged, indicating moisturization of the skin.

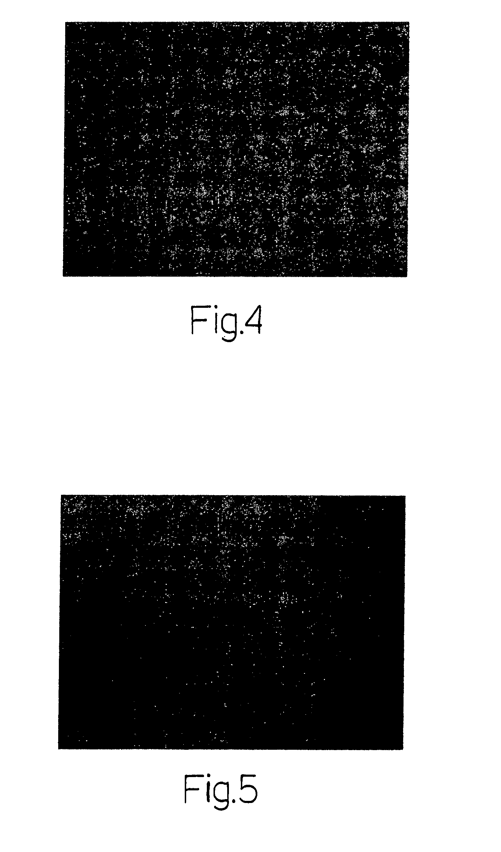

This is also confirmed by dermatoscope image readings (Figures 4, 5).

In fact, of the higher-intensity treated group, the ultrasound-treated hand images (Figure 5) proved "smoother" than the untreated-hand images (Figure 4) (after irradiation of 1 minute in 112 out of 132 cases; 15 minutes in 102 out of 132 cases; and 60 minutes in 78 out of 132 cases).

Testing shows the best moisturizing results are achieved with an "ultrasound dosage" ranging between 5 J/cm2 and 400 J/cm2, and preferably of 50 J/cm2; with cosmetic treatment of 2 to 10 minutes; and with ultrasonic wave frequencies ranging between 20 kHz and 50 kHz, and preferably of 40 kHz.

Duty cycle

The ultrasound energy necessary to administer the selected "ultrasound dosage" may be supplied continuously or intermittently (alternating on-off supply of the energy defining the so-called "duty cycle").

The "duty cycle (DC)" is defined by the following quantity:

$$DC = 100 \ ON/OFF$$

where "ON" is the supply time per second, and "OFF" the non-supply time per second.

The user may select DCs of 50% to 100%, with 10% intervals.

Each individual supply lasts 100 ms (0.1 s).

As the duty cycle is reduced, an automatic device increases instantaneous intensity to ensure the same mean intensity with time, regardless of the chosen duty cycle.

Intermittent, as opposed to continuous, energy supply, in fact, improves ultrasound efficiency in terms of induced skin permeability.

Conclusions

The above observations can be summed up as follows:

　　1. Echograph analysis shows subcorneum moisturization in the form of :

　　　　a. echographic fading of the stratum corneum (water) ;
　　　　b. increased subcorneum thickness;

　　2. microscope analysis shows subcorneum moisturization in the form of tensioning of skin folds and a visible reduction in wrinkling of the skin;
　　3. the effectiveness of the treatment is shown by a significant number of volunteers actually being able to determine the treated skin portion, and with no particular side-effects, except in very few cases, who reported a slight tingling effect following treatment;
　　4. measurements performed on a number of volunteers (5) would indicate a visible-effectiveness threshold of 3 to 4 minutes.

Moreover, echographically, moisturizing creams fail to provide for moisturizing, or altering the exogenous structure of, the subcorneum, and in this respect have a purely "aesthetic" effect. A combination of moisturizing cream and the cosmetic treatment according to the present invention is therefore desirable to improve moisturization by penetration of water and cream, and also ensure better and longer-lasting results through use of the cream.

The main advantage of the cosmetic treatment according to the present invention substantially lies in permitting effective moisturization of the skin using only water, i.e. no moisturizing creams or other substances, unless desired.

**Claims**

1. A cosmetic treatment for moisturizing human skin, the cosmetic treatment being **characterized by** subjecting the skin to an "ultrasound dosage" ranging between 5 J/cm2 and 400 J/cm2.

2. A cosmetic treatment as claimed in Claim 1, **characterized in that** the "ultrasound dosage" is preferably 50 J/cm2.

3. A cosmetic treatment as claimed in either of the foregoing Claims, **characterized by** lasting from 2 to 10 minutes.

4. A cosmetic treatment as claimed in any one of the foregoing Claims, **characterized in that** the ultrasonic waves are of 20 kHz to 50 kHz frequency.

5. A cosmetic treatment as claimed in Claim 4, **characterized in that** the ultrasonic waves are preferably of 40 kHz frequency.

6. A cosmetic treatment as claimed in any one of the foregoing Claims, **characterized by** comprising a "duty cycle" for supplying the ultrasound energy necessary to administer the selected "ultrasound dosage".

7. A cosmetic treatment as claimed in Claim 6, **characterized in that**,
   if "duty cycle (DC)" is defined by the following quantity:

$$DC = 100 \ ON/OFF$$

   where "ON" is the supply time per second, and "OFF" the non-supply time per second,
   the user may select a "duty cycle" value of 50% to 100%, with 10% intervals.

8. A cosmetic treatment as claimed in Claim 7, **characterized in that** each individual supply lasts 100 ms (0.1 s).

9. A cosmetic treatment as claimed in Claim 8, **characterized in that**, as the "duty cycle" is reduced, there is a corresponding increase in instantaneous ultrasound energy intensity, to ensure the same mean intensity with time, regardless of the selected duty cycle.

10. A cosmetic apparatus (100), **characterized by** implementing a cosmetic treatment as claimed in any one of the foregoing Claims.

11. An apparatus (100) as claimed in Claim 10, **characterized by** comprising a tub (108) having at least one emitting transducer (104).

Fig.1

Fig.2

Fig.3

Fig.4

Fig.5

**European Patent Office**

## PARTIAL EUROPEAN SEARCH REPORT

which under Rule 45 of the European Patent Convention shall be considered, for the purposes of subsequent proceedings, as the European search report

**Application Number**

EP 05 42 5883

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 048 520 A (VAGO ET AL) 17 September 1991 (1991-09-17) * column 2, line 47 * * column 1, lines 10-17 * * column 15, lines 10,15,16,32-41; figure 2; tables 1,2 * * column 7, lines 56-64 * * column 4, lines 20,21 * ----- | 10,11 | A61H33/00 A61N7/00 |
| X | US 5 339 804 A (KEMP ET AL) 23 August 1994 (1994-08-23) * column 1, lines 11,47,55-68; claims 2,3; figure 1 * * column 3, lines 5-13 * ----- | 10,11 | |
| X | US 3 867 929 A (JOYNER ET AL) 25 February 1975 (1975-02-25) * column 4, lines 20-22; figure 2; table 2 * ----- | 10,11 | |
| A | EP 0 301 162 A (AUTENRIETH, WILLI) 1 February 1989 (1989-02-01) * column 1, lines 1-8; claim 1 * * column 2, line 8; figure 1 * ----- | 10,11 | **TECHNICAL FIELDS SEARCHED (IPC)** A61H A61N |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 30 March 2006 | Anscombe, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent

Office

**PARTIAL EUROPEAN SEARCH REPORT**

Application Number

EP 05 42 5883

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | EP 0 651 987 A (TEUCO GUZZINI S.P.A; TEUCO GUZZINI S.R.L) 10 May 1995 (1995-05-10)<br>* column 4, lines 23-36; figure 1 *<br>----- | 10,11 | |
| | | | **TECHNICAL FIELDS SEARCHED** (IPC) |

EPO FORM 1503 03.82 (P04C10)

**INCOMPLETE SEARCH
SHEET C**

Application Number

EP 05 42 5883

Claim(s) not searched:
        1-9

Reason for the limitation of the search (non-patentable invention(s)):

It is known that abnormally dry skin (xeroderma) can require treatment for clinical reasons. Therefore any method which moisturises the human skin is a prophylactic treatment for abnormally dry skin (xeroderma). Therefore the subject matter of method claims 1-9 is not patentable (Art. 52(4) EPC) because the subject matter describes a method of treatment of the human body which has and inevitable and inextricably linked therapeutic, in this case prophylactic, effect (Guidelines C-IV,4.2.1). The fact that from the wording of the claim the intention of the treatment is to provide a "cosmetic" effect does not prevent the subject matter of claims 1-9 from being excluded from patentability on the grounds that it is therapeutic (T1172/03).

**ANNEX TO THE EUROPEAN SEARCH REPORT**

**ON EUROPEAN PATENT APPLICATION NO.**                    EP 05 42 5883

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2006

| Patent document cited in search report | | Publication date | Patent family member(s) | | Publication date |
|---|---|---|---|---|---|
| US 5048520 | A | 17-09-1991 | CA | 1330668 C | 12-07-1994 |
| | | | DK | 151589 A | 01-10-1989 |
| | | | FI | 891461 A | 01-10-1989 |
| | | | JP | 1923538 C | 25-04-1995 |
| | | | JP | 2203859 A | 13-08-1990 |
| | | | JP | 6049054 B | 29-06-1994 |
| | | | JP | 7000469 A | 06-01-1995 |
| | | | NO | 891321 A | 02-10-1989 |
| US 5339804 | A | 23-08-1994 | CA | 2100728 A1 | 17-01-1995 |
| US 3867929 | A | 25-02-1975 | GB | 1297369 A | 22-11-1972 |
| EP 0301162 | A | 01-02-1989 | DE | 8714883 U1 | 04-02-1988 |
| EP 0651987 | A | 10-05-1995 | IT | MI932341 A1 | 04-05-1995 |

EPO FORM P0459

For more details about this annex : see Official Journal of the European Patent Office, No. 12/82